# EUROPÄISCHE PATENTSCHRIFT

(11) **EP 2 442 917 B1**
(45) Veröffentlichungstag und Bekanntmachung des Hinweises auf die Patenterteilung: **04.11.2015**
(21) Anmeldenummer: 10730117.8
(22) Anmeldetag: 16.06.2010
(51) Int. Cl.: B05D 7/00, B05D 7/20, B05C 3/00

(54) **VERFAHREN UND VORRICHTUNG ZUR BESCHICHTUNG VON KATHETERN ODER BALLONKATHETERN**
METHOD AND DEVICE FOR COATING CATHETERS OR BALLOON CATHETERS
PROCÉDÉ ET DISPOSITIF D'ENDUCTION DE CATHÉTERS SIMPLES OU À BALLONNET

(30) Priorität: 17.06.2009 DE 102009025638
(43) Veröffentlichungstag der Anmeldung: 25.04.2012
(73) Patentinhaber: DOT GmbH, 18059 Rostock (DE)
(72) Erfinder: NEUMANN, Hans-Georg, 18146 Rostock (DE); BUHRMEISTER, Mischa, 18059 Papendorf (DE)
(74) Vertreter: Prinz & Partner mbB
(86) Internationale Anmeldenummer: PCT/EP2010/058493
(87) Internationale Veröffentlichungsnummer: WO 2010/146096

(56) Entgegenhaltungen:
- US-B2- 6 406 754

## Beschreibung

Die Erfindung betrifft ein Verfahren und eine Vorrichtung zur gezielten Beschichtung von Kathetern oder Ballonkathetern.

### Stand der Technik

Die Behandlung von Stenosen erfolgt durch das Setzen von Stents und/oder durch die Verwendung sogenannter Ballonkatheter, um die entsprechenden Gefäße wieder aufzuweiten. In den letzten Jahren sind hierbei bedeutende Erfolge erzielt worden. In vielen Fällen kann dadurch eine Erweiterung des Gefäßlumens auf über 90% des Wertes vor Einsetzen der Verengung erzielt werden.

Bei zahlreichen Patienten kam es jedoch in der Vergangenheit nach wenigen Monaten zu einer erneuten Verengung (Restenose). Dies ist hauptsächlich eine Folge der durch die gewaltsame Aufdehnung der Gefäßwände ausgelösten übermäßigen Proliferation insbesondere der glatten Muskelzellen. Nach Abheilung der Verletzung kommt deren Proliferation nicht sofort zum Stillstand und führt so häufig zu einer Restenose. Diesem Effekt kann man durch die Beschichtung der Stents bzw. der Ballonkatheter mit einem Anti-Restenose-Wirkstoff entgegenwirken.

Die Ballonkatheter und Stents müssen in der Regel einen längeren Weg innerhalb der Gefäße zurücklegen, bevor sie an den Ort der Gefäßverengung gelangen. Hier stellt sich das Problem, eine vorzeitige Ablösung der Wirkstoffe vor Erreichen des Zielortes zu verhindern und zu gewährleisten, dass der Wirkstoff in vordefinierter Menge am Ort der Gefäßverengung zur Verfügung steht.

Bei der alleinigen Verwendung von Ballonkathetern kommt erschwerend hinzu, dass die Kontaktzeit mit der Gefäßwand bei der Aufdehnung des Ballons nur einige Sekunden bis wenige Minuten beträgt. Es ist also notwendig, die vorhandene Kontaktfläche durch eine gleichmäßige Beschichtung möglichst vollständig zu nutzen und die Wirkstoffe mit einer hohen Bioverfügbarkeit bereitzustellen.

Die Stenose von Arterien ist oft mit massiver Verkalkung verbunden. Die betroffenen Gefäße lassen sich nur durch sehr hohen Druck wieder auf ihr ursprüngliches Lumen aufweiten. Zu diesem Zweck wird ein druckresistenter Ballon eingeführt, der sich durch Dilatation zu einem festen Zylinder formt, welcher eng an der Gefäßwand anliegt und die außen aufgetragenen Wirkstoffe mit dem entsprechend hohen Druck an die Gefäßwände presst.

Mit der gleichen Methode kann auch eine lokale Arzneimittelgabe erfolgen, ohne dass eine Aufweitung der Gefäße notwendig ist. Beispiele lassen sich bei Veränderungen der Gefäßwand finden, die nicht mit einer Stenose verbunden sind (z.B. vulnerable Plaques, aufgelagerte Thromben). Andere Beispiele sind die Behandlung von Gefäßen mit mechanischen Mitteln oder mit thermischen Verfahren. Eine Überdehnung und eine damit verbundene Verletzung der Gefäßwände sind in diesen Fällen nicht erwünscht. Daher liegen die verwendeten Ballons auch nicht vollständig an den unregelmäßigen Gefäßwänden an und die Wirkstoffe werden mit geringerem Druck an diese gepresst.

In zahlreichen Veröffentlichungen werden Lösungen vorgeschlagen, die die vorzeitige Ablösung der Wirkstoffe bei Stents verhindern sollen. Eine Möglichkeit besteht darin, den Wirkstoff in kleinen Kavitäten unterzubringen und durch eine Schutzbeschichtung die frühzeitige Ablösung zu verhindern (US 2004/0071861 A, WO 2003/035131 A). Ebenso wird die Möglichkeit beschrieben, über der Wirkstoffschicht eine unelastische Deckschicht aufzubringen, die bei der Expansion aufbricht (WO 2000/45744 A1). Im Gegensatz dazu beschreibt DE 102007010354 A1 eine Kombination von Wirkstoffschicht und darüber liegender resorbierbarer Opferschicht.

Auch bei Ballonkathetern sind Beschichtungen mit Wirkstoffen beschrieben worden. Diese sind jedoch aufgrund der größeren Oberfläche und der gefalteten Struktur schwerer zu realisieren. Darüber hinaus ist bedingt durch die kurze Kontaktzeit eine sofortige Abgabe der Wirkstoffe vom Katheter an die Gefäßwände notwendig.

Die Möglichkeit, auch bei dem kurzen Kontakt der Ballonkatheter mit den Blutgefäßen zuverlässig eine Restenose zu verhindern, wurde erstmals in WO 2002/076509 A2 offenbart. Dazu wird ein Ballonkatheter beschrieben, der bei Kontakt mit der Gefäßwand den Wirkstoff sofort in bioverfügbarer Form freigibt.

In zahlreichen Dokumenten des Standes der Technik werden Wirkstoffe und komplexe Kombinationen aus verschiedensten Materialien beschrieben, die sich für eine Beschichtung von Stents und/oder Ballonkathetern eignen.

In EP 0519063 B1 wird die Möglichkeit offenbart, einen zunächst expandierten und danach wieder deflatierten Faltenballon mit Mikrokapseln zu beschichten, in denen ein pharmazeutischer Wirkstoff eingeschlossen sein kann. Ein Nachteil dieser Ausführungsform besteht darin, dass der größte Teil der Mikrokapseln beim Einführen des Ballonkatheters in ein Gefäß von der Ballonoberfläche gelöst wird, und nur die Kapseln in den Falten an den Bestimmungsort gelangen. Die Menge der bei der Expansion zur Verfügung stehenden Mikrokapseln, und damit die abgegebene Wirkstoffmenge, ist also nicht bekannt.

WO 2007/090385 A2 offenbart eine Möglichkeit, gezielt die Falten eines Ballonkatheters mit einem Wirkstoff zu versehen. Bei dieser Ausführungsform gelangt der gesamte Wirkstoffvorrat an den Bestimmungsort.

In den beiden vorgenannten Ausführungen (EP 0519063 B1 und WO 2007/090385 A2) wird der Wirkstoff nur über einen Teil der Ballonoberfläche an die Gefäßwände übertragen. Auf diese Weise kann nicht sicher gestellt werden, dass der Wirkstoff in der notwendigen Menge alle betroffenen Stellen der Gefäßwand erreicht.

In WO 2004/006976 A werden lipophile Arzneimittel durch Sprühen, Tauchen oder Aufsaugen auf eine strukturierte, insbesondere raue, Oberfläche des expandierten Ballons aufgetragen. Durch eine hydrophile Schicht zwischen dem Arzneimittel und der Ballonoberfläche soll die Ablösung des Wirkstoffes erleichtert werden.

Die meisten beschriebenen Lösungen zur Beschichtung von Kathetern und Ballonkathetern gehen von einem die Freisetzung der Wirkstoffe verzögernden Träger aus. Die Kontaktzeit zwischen Ballonoberfläche und umgebendem Gewebe ist dabei aber relativ kurz.

DE 102007036685 A1 offenbart dagegen die Beschichtung eines Ballonkatheters derart, dass mindestens ein Wirkstoff sofort freigesetzt wird. Dabei werden verschiedenste Zusammensetzungen aus Wirkstoffen und Transportvermittlern dargestellt.

In WO 2008/086794 A2 wird allgemein die Beschichtung von Katheterballons mithilfe einer Volumenmesseinrichtung und einer Abgabevorrichtung offenbart. Dabei kommen sehr viele verschiedene Verfahren für die Beschichtung zum Einsatz. Es ist jedoch bekannt, dass nicht alle dort beschriebenen Verfahren zu einer Oberfläche mit gleichen qualitativen Eigenschaften führen.

In US 6,322,847 B1 wird eine Möglichkeit offenbart, bei der Beschichtung entstehende überschüssige Anteile mit Hilfe eines Gasstrahls zu entfernen. Es ist allerdings vorteilhafter, derartige überschüssige Anteile schon während der Beschichtung zu vermeiden und so eine nachträgliche Behandlung überflüssig zu machen. Die Trocknung der Beschichtung während der Behandlung mit dem Gasstrahl lässt sich nicht vollständig vermeiden. Daher ist die Kalibrierung des Systems sehr aufwändig, denn einerseits kann ein zu schwacher Gasstrom die überschüssigen Anteile der härter werdenden Schicht nicht mehr entfernen und andererseits kann ein zu starker Gasstrom eine Beschädigung der Schicht derart erzeugen, dass das zu beschichtende Objekt nicht mehr vollständig bedeckt ist.

US 2006/0029720 A1 offenbart eine Methode, ein Medizinprodukt zu beschichten indem die Beschichtungslösung auf das obere Ende des senkrecht aufgestellten Medizinproduktes aufgebracht wird. Die Verteilung erfolgt dann durch Fließprozesse auf Grund der Schwerkraft. Ein Vorteil der Erfindung ist die gute Möglichkeit der Automatisierung. Bei Medizinprodukten, insbesondere Stents, ist eine gleichmäßige Beschichtung und insbesondere eine sehr gleichmäßige Verteilung der enthaltenden Wirkstoffe über die Oberfläche von großer Bedeutung. Die gleichmäßige Verteilung der Beschichtungslösung stellt bei dieser Methode, jedoch ein Problem dar. Das gilt insbesondere für stark strukturierte Objekte wie Stents oder Ballonkatheter. Die Bildung von Überständen und Tropfen lässt sich bei der Fließtechnik praktisch nicht verhindern und es müssen zusätzliche Verfahren, wie z.B. nach US 6,322,847 B1 zum Einsatz kommen.

US 6,406,754 B2 und US 6,254,921 B1 beschreiben ein Gerät und eine Methode, zur Beschichtung eines röhrenförmigen oder drahtähnlichen Medizinproduktes mit der Möglichkeit, Bereiche, die keine Beschichtung erhalten sollen, auszulassen. Mit diesem Verfahren ist es auch möglich, auf verschiedene Bereiche des Medizinproduktes verschiedene Beschichtungen aufzubringen. Zu diesem Zweck wird eine Kammer verwendet, die an den Enden das Medizinprodukt so umschließt, das keine Beschichtungslösung austreten kann. Dabei kann die Beschichtung durch Zu- und Abführung der Beschichtungslösung in der Kammer oder durch eine Relativbewegung von Kammer und Medizinprodukt erfolgen. Diese Lösung hat gleich mehrere Nachteile. Der dichte Abschluss der Kammer an den Enden ist technologisch sehr aufwändig, da viele dieser Produkte entweder stark strukturiert sind, z.B. Stents, oder von sich aus Falten aufweisen wie z.B. Ballonkatheter. Bei einer Relativbewegung von Kammer und Medizinprodukt können durch den dichten Abschluss die neue Beschichtung, schon vorhandene Beschichtungen oder sogar das Medizinprodukt selbst beschädigt werden. Ebenso stellen in der Kammer verbleibende Restmengen und Beschädigungen der Beschichtung an den Abdichtungsstellen bei der Öffnung der Kammer ein Problem dar.

In US 2007/0128343 A1 wird eine Möglichkeit offenbart, mit Hilfe von zwei Sprühköpfen zwei verschiedene Beschichtungslösungen aufgebracht werden können. Ein Sprühverfahren hat mehrere Nachteile, die von der vorliegenden Veröffentlichung nicht überwunden werden. Für eine gleichmäßige Beschichtung müssen die Sprühköpfe und das zu beschichtende Objekt nicht nur relativ der Längsachse bewegt werden, sondern es muss auch eine Rotationsbewegung des Objektes oder eine entsprechende Bewegung der Sprühköpfe erfolgen. Die genaue Menge der auf den Objekten aufgebrachten Substanzen lässt sich nur schwer garantieren, da immer ein Teil der Substanzen beim Sprühen nicht auf dem zu beschichtenden Objekt ankommt. Gerade bei medizinisch wirksamen Komponenten ist dies jedoch von großer Bedeutung.

Mögliche Zusammensetzungen von Beschichtungslösungen werden in den Dokumenten zum Stand der Technik ausführlich beschrieben. Die Verfahren zur Beschichtung werden in erster Linie unter den Gesichtspunkten der gezielten Befüllung der Falten des Katheterballons und/oder der Aufbringung einer bestimmten Menge der Beschichtungslösung dargestellt.

Ebenso werden viele Möglichkeiten der Gestaltung der Oberfläche des Ballons, sei es durch Strukturierung oder durch eine zusätzliche Behandlung, offenbart.

Den Dokumenten zum Stand der Technik ist gemeinsam, dass der Einfluss der Beschichtungsmethode auf die Struktur und die Qualität der Oberfläche nicht ausreichend betrachtet wird. Ebenso werden die Umgebungsbedingungen und weitere Prozessparameter, die einen entscheidenden Einfluss auf die qualitativen Eigenschaften der Oberflächen haben, nur ungenügend dargestellt.

### Darstellung der Erfindung

Aufgabe der Erfindung ist es, ein Verfahren und eine Vorrichtung zu entwickeln, welche eine kontinuierliche und gleichmäßige Beschichtung von Kathetern und Ballonkathetern ermöglichen. Verwendet werden dabei handelsübliche Katheter ohne eine notwendige spezielle Vorbehandlung.

Das erfindungsgemäße Verfahren zur Beschichtung von Kathetern oder Ballonkathetern ist dadurch gekennzeichnet,
a. dass der Katheter mit einer Vorrichtung, welche eine abgemessene Menge einer Beschichtungslösung enthält, mit einem gleichbleibenden Abstand vollständig oder annähernd vollständig derart radial umschlossen wird,
b. dass die Beschichtungslösung den Raum zwischen dem Katheter und der Vorrichtung vollständig ausfüllt und so den Katheter auf einem axialen Teilabschnitt vollständig radial umhüllt,
c. dass die Vorrichtung in axialer Richtung mehrfach über die Oberfläche des Katheters bewegt wird und
d. dass zwischen den einzelnen Beschichtungsvorgängen eine gezielte teilweise Trocknung der aufgetragenen Beschichtungslösung erfolgt.

Anschließend erfolgt eine vollständige Trocknung nach dem Stand der Technik.

Zusätzlich kann eine Gasströmung durch eine Einrichtung zur Erzeugung einer Gasströmung entgegen der Flussrichtung der Beschichtungslösung erzeugt werden, die eine Anreicherung der Beschichtungslösung und/oder eine Tropfenbildung z.B. an den Endpunkten des Katheters verhindert. Besonders vorteilhaft ist dieser Verfahrensschritt bei Ballonkathetern an den Enden des Ballons anzuwenden.

Die Gasströmung besteht aus einem Gas oder Gasgemisch, erfolgt gleichmäßig oder mit zeitlichen Änderungen in der Richtung und der Stärke und kann zeitlich veränderlich thermisch beeinflusst werden.

Sowohl die teilweise Trocknung der Beschichtung zwischen den Beschichtungsvorgängen und als auch die endgültige Trocknung der Beschichtung kann durch einen zusätzlichen zweiten Luftstrom unterstützt werden. Über die Temperatur und die Stärke des Luftstroms kann der Grad der Trocknung in einer bestimmten Zeiteinheit eingestellt und so an die Dauer eines Beschichtungsvorganges angepasst werden.

Alternativ kann der Trocknungsprozess auch durch Veränderung der Druckverhältnisse unterstützt werden. Ebenso sind andere dem Fachmann geläufige Verfahren anwendbar.

Die Beschichtung des Katheters einschließlich der teilweisen und vollständigen Trocknung kann mehrmals wiederholt werden. Dabei ist ein Wechsel der Beschichtungslösung möglich.

In einer weiteren Ausführung wird die axiale Bewegung durch Bewegung des Katheters, durch Bewegung der umschließenden Vorrichtung oder durch Bewegung beider erzeugt. Der Katheter ist feststehend oder wird um seine Längsachse gedreht.

Die Beschichtungslösung enthält mindestens einen pharmakologischen Wirkstoff, wahlweise keinen, einen oder mehrere Zusatzstoffe und mindestens ein Lösungsmittel in einer definierten Zusammensetzung. Sie wird kontinuierlich oder diskontinuierlich definiert erneuert.

Wird ein Ballonkatheter beschichtet, entfaltet sich dieser durch Erwärmung vor und/oder während des Beschichtungsvorganges teilweise. Die Erwärmung erfolgt gleichmäßig, mit räumlicher Verteilung und/oder zeitlicher Veränderung durch Erwärmung der Umgebung, durch Erwärmung der Haltevorrichtung, durch Strahlungswärme oder durch einen erwärmten Gasstrom über den Ballonkatheter. Durch diese teilweise Entfaltung gelangt die Beschichtungslösung auch auf die Innenseite der Falten und ein in der Beschichtungslösung enthaltener Wirkstoff bei der Anwendung des Ballonkatheters auf der gesamten Oberfläche seine Wirkung entfalten kann.

Die erfindungsgemäße Vorrichtung umschließt den Katheter vollständig oder teilweise radial, in der Art, dass zwischen Katheter und Vorrichtung ein gleichbleibender radialer Abstand entsteht, der groß genug ist um eine Berührung zwischen der Oberfläche des Katheters und der Vorrichtung zuverlässig zu verhindern aber so klein, dass eine für die Beschichtung minimal notwendige Menge der Beschichtungslösung den sich bildenden Raum ausfüllen kann. In axialer Richtung ist die Ausdehnung der Vorrichtung zur Beschichtung im Verhältnis zur Länge des zu beschichtenden Objektes relativ klein.

Die umschließende Vorrichtung ist als Ring, Rohr oder Wendel ausgeführt. Sie kann einen schmalen Schlitz aufweisen und besteht aus metallischen und/oder nicht metallischen Materialien. Die Oberfläche der umschließenden Vorrichtung ist beschichtet und/oder mit einer zusätzlichen Struktur versehen.

Der Katheter wird mit Hilfe einer Halterung, auf die der Katheter geschoben wird, in einer Haltevorrichtung fixiert. Die Vorrichtung wird mehrfach axial über den Katheter bewegt. An den Enden des Katheters werden Gasströmungen erzeugt, die eine Anreicherung der Beschichtungslösung und/oder eine Tropfenbildung verhindern. Ein erwärmter Gasstrom unterstützt die teilweise Trocknung zwischen den Beschichtungsvorgängen und die endgültige Trocknung.

Das erfindungsgemäße Verfahren eignet sich nicht nur zur Beschichtung von Kathetern und Ballonkathetern, sondern auch zur Beschichtung von allen anderen Kathetern und anderen, vorzugsweise walzenförmigen, medizinischen Geräten mit relativ gleich bleibendem Durchmesser.

Vorzugsweise wird die in der Vorrichtung enthaltene Beschichtungslösung während einer Beschichtung, die aus mehreren Beschichtungs- und Trocknungsvorgängen bestehen kann, vollständig verbraucht. Da bei mehreren aufeinanderfolgenden Beschichtungen die Benetzung der Vorrichtung mit der Beschichtungslösung zum Beginn der Beschichtung der Benetzung am Ende der Beschichtung entspricht kann aus der Menge der verbrauchten Beschichtungslösung sehr genau die erhaltene Beschichtung ermittelt werden. Dies ist bei den aus dem Stand der Technik bekannten Tauch- und Sprühverfahren nicht der Fall. Für eine größere Schichtdicke kann die Vorrichtung auch während des Beschichtungsvorganges mit einer ebenfalls genau dosierten Menge Beschichtungslösung neu gefüllt werden.

Durch die teilweise Trocknung der Beschichtungslösung zwischen den einzelnen Beschichtungsvorgängen wird erreicht, dass die schon vorhandene Beschichtung beim nächsten Vorgang nicht zusammengeschoben oder zerstört wird. Dadurch wird eine sehr gleichmäßige Beschichtung, bei in weiten Bereichen wählbarer Schichtdicke, erreicht, wie sie mit anderen Verfahren (z.B. US 2006/0029720 A1) nicht möglich ist. Eine nachträgliche Beseitigung von überschüssiger Beschichtungslösung (z.B. US 6,322,847 B1) ist nicht notwendig.

Durch den Grad der teilweisen Trocknung zwischen den Beschichtungsvorgängen kann eingestellt werden, ob die Schichten aus den einzelnen Beschichtungsvorgängen sich wieder zu einer einzelnen Schicht verbinden (geringer Grad der Trocknung) oder ob die einzelnen Schichten im Querschnitt weitestgehend erhalten bleiben.

Ebenso ist eine Wiederholung des gesamten Vorganges, auch mit einer anderen Beschichtungslösung, möglich. Dabei hängt es von den verwendeten Beschichtungslösungen und der angestrebten Gesamtschicht ab, ob in der Zwischenzeit eine teilweise oder eine vollständige Trocknung vorteilhaft ist.

### Kurze Beschreibung der Abbildungen

Die Erfindung wird anhand von Zeichnungen näher erläutert. Hierzu zeigt
Figur 1 die erfindungsgemäße Lösung mit einer Beschichtungseinrichtung, die den Katheter nicht vollständig umhüllt, und
Figur 2 die erfindungsgemäße Lösung mit Haltevorrichtung und Einrichtung zur Erzeugung einer Gasströmung.

### Ausführung der Erfindung

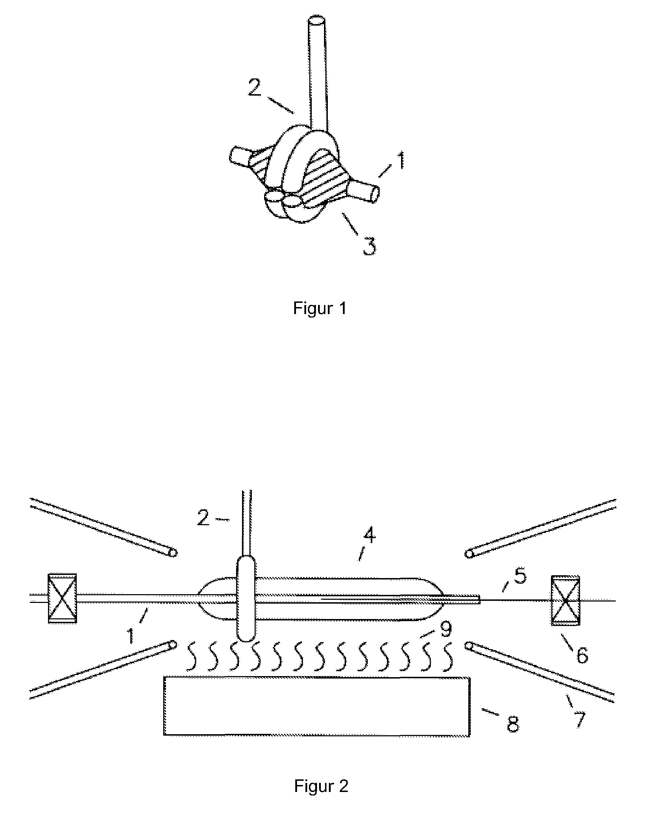

Die Vorrichtung 2 entsprechend Figur 1 zur Beschichtung eines Katheters umschließt den Katheter 1 vollständig oder teilweise radial. Gezeigt wird hier eine nicht vollständige Umhüllung des Katheters 1. Zwischen Katheter 1 und Vorrichtung 2 ist ein gleich bleibend definierter Abstand, so dass die inneren radialen Abmessungen der Vorrichtung 2 größer sind als z.B. der Durchmesser eines gefalteten Ballons. Die Vorrichtung 2 kann unterschiedliche Ausführungsformen besitzen, beispielsweise als Ring, Rohr, Wendel oder eine andere geeignete Form. Die Vorrichtung 2 kann aus metallischen und nicht metallischen Materialien bestehen. Bezüglich der axialen Ausdehnung des Katheterballons ist die Breite der Vorrichtung 2 gering (Bild 2).

Die Oberfläche der umschließenden Vorrichtung 2, insbesondere der Innenseite, kann beschichtet und/oder mit einer zusätzlichen Struktur versehen sein.

In die umschließende Vorrichtung 2 wird eine definierte Menge der Beschichtungslösung 3 gefüllt, die während der Beschichtung durch eine ebenfalls definierte Menge erneuert wird. Die Erneuerung kann kontinuierlich oder diskontinuierlich erfolgen. Die Beschichtungslösung 3 enthält mindestens einen pharmakologischen Wirkstoff, wahlweise einen oder mehrere Zusatzstoffe und mindestens ein Lösungsmittel in einer definierten Zusammensetzung.

Die Beschichtungslösung 3 umhüllt den Katheter 1 vollständig radial entsprechend der Abmessungen der umschließenden Vorrichtung 2 und wird in axialer Richtung über die Oberfläche des Katheters 1 bewegt, wobei eine definierte Menge der Beschichtungslösung 3 aufgebracht wird. Die axiale Bewegung wird entweder dadurch erzeugt, dass sich der Katheter 1 durch die umschließende Vorrichtung 2 bewegt oder dass diese über den Katheter 1 bewegt wird. Eine weitere Möglichkeit besteht darin, dass sowohl der Katheter 1 als auch die umschließende Vorrichtung 2 bewegt werden. Ebenso kann der Katheter 1 um seine Längsachse gedreht werden oder feststehend sein.

Schon während der Beschichtung findet die teilweise Trocknung statt. Dieser Prozess kann durch Erwärmung beschleunigt werden. In dem Beispiel wird die teilweise Trocknung zwischen den Beschichtungsvorgängen durch erwärmte Luft 9, die einem Gebläse 8 oder einer anderen geeigneten Vorrichtung, entströmt, beschleunigt.

Die umschließende Vorrichtung 2 kann mehrmals über den Katheter 1 bewegt werden, wobei der Ballon des Ballonkatheters vollständig mit einer Schicht versehen wird. Durch das wiederholte Antrocknen und Anfeuchten der Schicht wird einerseits die Zerstörung der schon vorhandenen Schicht beim nächsten Vorgang verhindert, andererseits bildet sich am Ende der Beschichtung eine sehr homogene einheitliche Schicht.

In einer Ausführungsform ist der Katheter 1 ein Ballonkatheter (Figur 2), wobei der Ballon des Ballonkatheters 4 beschichtet wird. Eine teilweise Inflation des gefalteten Ballons erfolgt durch Erwärmung. Für die Erwärmung des Ballonkatheters können mehrere Faktoren sorgen: die Erwärmung der Umgebung, die Erwärmung der Haltevorrichtung 6, Strahlungswärme, ein erwärmter Gasstrom über dem Ballonkatheter oder eine andere zur Erwärmung benutzbare Methode. Die Erwärmung kann gleichmäßig, mit räumlicher Verteilung und/oder zeitlicher Veränderung erfolgen.

Zur Gewährleistung einer homogenen Beschichtung wird strömendes Gas entgegen der Fließrichtung der Beschichtungslösung durch eine Einrichtung zur Erzeugung einer Gasströmung 7 eingesetzt. Der Gasstrom erfolgt gleichmäßig oder mit zeitlichen Änderungen in Richtung und Stärke.

Durch die Vorrichtung 2 werden Schichten mit großer Gleichmäßigkeit erzeugt, bei die aufgetragene Wirkstoffmenge sehr genau reproduzierbar festgelegt werden kann.

### Beispiel 1 - Beschichtung des Ballons eines Ballonkatheters mit einem pharmakologischen Wirkstoff und einem Bindemittel

Es wird eine Lösung aus dem pharmakologischen Wirkstoff, z.B. einem Mittel zur Verhinderung von Restenosen, einem Bindemittel, z.B. Shellack, und einem Lösungsmittel, vorzugsweise ein leicht flüchtiger Alkohol, nach dem Stand der Technik hergestellt.

Die Katheter 1 der verwendeten Ballonkatheter haben die Form von Röhren, die am vorderen Ende eine Öffnung haben. In diese Öffnung wird ein dünner Draht 5 eingeführt. Anschließend wird der Katheter 1 so in die Beschichtungseinrichtung eingespannt, dass sich der Ballon zwischen den beiden Haltevorrichtungen 6 befindet.

Die Umgebung des Ballons wird mit Heißluft 9 aus einem Gebläse 8 erwärmt. Es kann auch jede andere aus dem Stand der Technik bekannte Methode verwendet werden. Dabei hängt die gewählte Temperatur von der Zusammensetzung der Beschichtungslösung 3 ab. Gleichzeitig richten sich durch die Erwärmung die Falten des Ballons leicht auf, wodurch eine gleichmäßige Beschichtung der ganzen Oberfläche möglich ist.

Von den beiden Enden der Haltevorrichtung 6 wird zur Gewährleistung einer homogenen Ballonbeschichtung zusätzlich je ein Gasstrom in Richtung Ballonmitte durch die Einrichtung zur Erzeugung einer Gasströmung 7 erzeugt.

Die umschließende Vorrichtung 2 ist in diesem Fall ein Ring, der nicht vollständig geschlossen ist, so dass er bei eingespanntem Ballonkatheder über den Katheter, über den Draht oder über den Ballon geschoben werden kann (Figur 1). Der Ring wird nun so platziert, dass der Ballon sich im Zentrum des Ringes befindet. Es wird eine genau bemessene Menge der Beschichtungslösung 3 in den Ring gegeben, so dass sich eine gleichmäßige dünne Schicht rings um den zu beschichtenden Ballon bildet. Der Ring wird gleichmäßig zwischen den beiden Enden des Ballons hin und her gefahren. Der gesamte Vorgang wird dabei über eine Kamera kontrolliert.

Durch die zugeführte Heißluft erfolgt eine teilweise Trocknung der Beschichtung, so dass bei jeder neuen Benetzung eine erneute Anlösung der schon angetrockneten Schicht erfolgt. So wird einerseits die Zerstörung der schon vorhandenen Schicht beim nächsten Vorgang verhindert, andererseits bildet sich am Ende der Beschichtung eine sehr homogene einheitliche Schicht.

Wenn die Lösung verbraucht ist, wird so lange immer wieder eine genau dosierte Menge der Beschichtungslösung 3 hinzugegeben, bis sich die angestrebte Menge des Wirkstoffes auf der Ballonoberfläche befindet. Die dazu notwendige Menge Beschichtungslösung 3 ergibt sich aus der gewählten Konzentration des Wirkstoffes in der Beschichtungslösung 3, der Oberfläche des Ballons im entfalteten Zustand und der angestrebten Menge des Wirkstoffes je Flächeneinheit.

Nach der Beschichtung wird der Ballon noch einige Minuten nachgetrocknet und nach dem bekannten Stand der Technik verpackt und sterilisiert.

### Beispiel 2 - Beschichtung eines Ballonkatheters mit einer hydrophilen Substanz

Es wird eine Lösung aus einer hydrophilen Substanz, z.B. Shellack, und einem Lösungsmittel, vorzugsweise ein leicht flüchtiger Alkohol, hergestellt. Die Lösung wird mit einer Dosiereinrichtung nach dem Stand der Technik in der für einen Beschichtungsvorgang notwendigen Menge abgefüllt. Die dazu notwendige Menge Beschichtungslösung 3 ergibt sich aus der gewählten Konzentration der hydrophilen Substanz in der Lösung, der Oberfläche des Ballons und der angestrebten Menge der Substanz je Flächeneinheit.

Die umschließende Vorrichtung 2 ist in diesem Beispiel ein kurzes vollständiges Rohrstück (Figur 2). Die Vorrichtung 2 zur Beschichtung wird von der Seite so über den Katheter 1 geschoben, dass dieser ungefähr im Zentrum des Rohrstücks liegt. Die Umgebung des Katheters 1 wird mit Heißluft erwärmt. Dabei hängt die gewählte Temperatur von der Zusammensetzung der Beschichtungslösung 3 ab. Das Rohstück wird dann gleichmäßig zwischen den beiden Enden des Ballons hin und her gefahren. Dabei wird so lange stetig die Beschichtungslösung 3 zugeführt, bis die vorher abgemessene Menge verbraucht ist. Der gesamte Vorgang wird dabei über eine Kamera kontrolliert.

Durch die zugeführte Heißluft erfolgt eine schnelle Trocknung, so dass bei jeder neuen Benetzung eine erneute Anlösung der schon angetrockneten Schicht erfolgt. Damit wird eine äußerst gleichmäßige Oberfläche erzeugt.

Nach der Beschichtung kann der Katheter noch nachgetrocknet werden und wird anschließend nach dem bekannten Stand der Technik verpackt und sterilisiert.

## Patentansprüche

1. Verfahren zur Beschichtung von Kathetern oder Ballonkathetern **dadurch gekennzeichnet,**
a. **dass** der Katheter (1) mit einer Vorrichtung (2), welche eine abgemessene Menge einer Beschichtungslösung (3) enthält, mit einem gleichbleibenden Abstand vollständig oder annähernd vollständig derart radial umschlossen wird,
b. **dass** die Beschichtungslösung den Raum zwischen dem Katheter (1) und der Vorrichtung (2) vollständig ausfüllt und so den Katheter (1) auf einem axialen Teilabschnitt vollständig radial umhüllt,
c. **dass** die Vorrichtung (2) in axialer Richtung mehrfach über die Oberfläche des Katheters (1) bewegt wird und
d. **dass** zwischen den einzelnen Beschichtungsvorgängen eine gezielte teilweise Trocknung der aufgetragenen Beschichtungslösung (3) erfolgt.

2. Verfahren nach Anspruch 1 **dadurch gekennzeichnet, dass** mit Hilfe einer zusätzlichen Einrichtung zur Erzeugung einer Gasströmung (7) eine Gasströmung entgegen der Flussrichtung der Beschichtungslösung (3) erzeugt wird, die eine Anreicherung der Beschichtungslösung (3) und/oder eine Tropfenbildung an den Endpunkten des Katheters oder des Ballonkatheters (1) verhindert.

3. Verfahren nach Anspruch 2 **dadurch gekennzeichnet, dass** die Gasströmung aus einem Gas oder Gasgemisch besteht, gleichmäßig oder mit zeitlichen Änderungen in der Richtung und der Stärke ausgeführt und zeitlich veränderlich thermisch beeinflusst wird.

4. Verfahren nach einem der Ansprüche 1 bis 3 **dadurch gekennzeichnet, dass** die teilweise Trocknung zwischen den Beschichtungsvorgängen und die endgültige Trocknung am Ende der Beschichtung durch Erwärmung in einem zusätzlichen Strom eines Gases oder eines Gasgemisches (9) beschleunigt werden.

5. Verfahren nach einem der Ansprüche 1 bis 4 **dadurch gekennzeichnet, dass** die teilweise Trocknung zwischen den Beschichtungsvorgängen und die endgültige Trocknung am Ende der Beschichtung dadurch beschleunigt wird, das die Beschichtung in einem Raum mit verändertem Druck erfolgt.

6. Verfahren nach einem der Ansprüche 1 bis 5 **dadurch gekennzeichnet, dass** die Beschichtung des Katheters (1) einschließlich der teilweisen und vollständigen Trocknung mehrmals wiederholt wird.

7. Verfahren nach einem der Ansprüche 1 bis 5 **dadurch gekennzeichnet, dass** ein Ballonkatheter beschichtet wird, der sich durch Erwärmung vor und/oder während des Beschichtungsvorganges teilweise entfaltet.

8. Verfahren nach Anspruch 7 **dadurch gekennzeichnet, dass** die Erwärmung gleichmäßig, mit räumlicher Verteilung und/oder zeitlicher Veränderung durch Erwärmung der Umgebung, durch Erwärmung der Haltevorrichtung, durch Strahlungswärme oder durch einen erwärmten Gasstrom über den Ballonkatheter erfolgt.

9. Verfahren nach einem der Ansprüche 1 bis 8 **dadurch gekennzeichnet, dass** die axiale Bewegung durch Bewegung des Katheters (1), durch Bewegung der umschließenden Vorrichtung (2) oder durch Bewegung beider erzeugt wird.

10. Verfahren nach einem der Ansprüche 1 bis 9 **dadurch gekennzeichnet, dass** der Katheter (1) feststehend ist oder um seine Längsachse gedreht wird.

11. Verfahren nach einem der Ansprüche 1 bis 10 **dadurch gekennzeichnet, dass** die Beschichtungslösung (3) mindestens einen pharmakologischen Wirkstoff, wahlweise keinen, einen oder mehrere Zusatzstoffe und mindestens ein Lösungsmittel in einer definierten Zusammensetzung enthält.

12. Verfahren nach einem der Ansprüche 1 bis 11 **dadurch gekennzeichnet, dass** die Beschichtungslösung (3) kontinuierlich oder diskontinuierlich definiert erneuert wird.

13. Vorrichtung zur Beschichtung von Kathetern oder Ballonkathetern **gekennzeichnet, dadurch**
a. **dass** die umschließende Vorrichtung (2) als Ring, Rohr oder Wendel ausgeführt ist,
b. **dass** der Katheter (1) mit Hilfe einer Halterung (5), auf die der Katheter (1) geschoben wird, in einer Haltevorrichtung (6) fixiert wird,
c. **dass** die Vorrichtung (2) mehrfach axial über den Katheter(1) bewegt wird,
d. **dass** mit einem Gebläse (8) ein erwärmter Gasstrom (9) erzeugt wird, der die teilweise Trocknung zwischen den Beschichtungsvorgängen und die endgültige Trocknung unterstützt und
e. **dass** an den Enden des Katheters (1) mit den Einrichtungen zur Erzeugung einer Gasströmung (7) Gasströmungen erzeugt werden, die eine Anreicherung der Beschichtungslösung und/oder eine Tropfenbildung verhindern.

14. Vorrichtung nach Anspruch 13, **dadurch gekennzeichnet, dass** die umschließende Vorrichtung (2) einen schmalen Schlitz aufweist.

15. Vorrichtung nach Anspruch 13 oder 14 **dadurch gekennzeichnet, dass** die umschließende Vorrichtung (2) aus metallischen und/oder nicht metallischen Materialien besteht.

16. Vorrichtung nach einem der Ansprüche 13 bis 15 **dadurch gekennzeichnet, dass**
die Oberfläche der umschließenden Vorrichtung (2) beschichtet und/oder mit einer zusätzlichen Struktur versehen ist.

17. Vorrichtung nach einem der Ansprüche 13 bis 16, **dadurch gekennzeichnet, dass**
die teilweise Trocknung zwischen den Beschichtungsvorgängen und die endgültige Trocknung dadurch unterstützt wird, dass die Beschichtung in einem Raum mit verändertem Druck stattfindet.

## Claims

1. A method of coating catheters or balloon catheters, **characterized in that**
a. the catheter (1) is completely or approximately completely radially surrounded at a constant distance with a device (2) which contains a measured quantity of a coating solution (3) such that
b. the coating solution completely fills the space between the catheter (1) and the device (2) and thus completely encloses the catheter (1) radially on an axial partial section;
c. **in that** the device (2) is moved across the surface of the catheter (1) a plurality of times in the axial direction, and
d. **in that** a targeted partial drying of the applied coating solution (3) is effected between the individual coating processes.

2. The method according to claim 1, **characterized in that**
with the aid of an additional means for generating a gas flow (7), a gas flow is generated opposite to the direction of flow of the coating solution (3) which prevents an enrichment of the coating solution (3) and/or a drop formation at the end points of the catheter or the balloon catheter (1).

3. The method according to claim 2, **characterized in that**
the gas flow consists of a gas or gas mixture, is carried out uniformly or with time variations in terms of direction and intensity, and is thermally influenced in a time-variable manner.

4. The method according to any of claims 1 to 3, **characterized in that**
the partial drying between the coating processes and the final drying at the end of the coating are accelerated by heating in an additional stream of a gas or a gas mixture (9).

5. The method according to any of claims 1 to 4, **characterized in that**
the partial drying between the coating processes and the final drying at the end of the coating are accelerated **in that** the coating is performed in a chamber with an altered pressure.

6. The method according to any of claims 1 to 5, **characterized in that**
the coating of the catheter (1) inclusive of the partial and complete drying is repeated a plurality of times.

7. The method according to any of claims 1 to 5, **characterized in that**
a balloon catheter is coated which partially unfolds by heating prior to and/or during the coating process.

8. The method according to claim 7, **characterized in that**
the heating is effected uniformly, with spatial distribution and/or time variation by heating the surroundings, by heating the holding device, by radiant heat, or by means of a heated gas stream across the balloon catheter.

9. The method according to any of claims 1 to 8, **characterized in that**
the axial movement is generated by moving the catheter (1), by moving the surrounding device (2), or by moving both.

10. The method according to any of claims 1 to 9, **characterized in that**
the catheter (1) is stationary or is rotated about its longitudinal axis.

11. The method according to any of claims 1 to 10, **characterized in that**
the coating solution (3) contains at least one pharmacological agent, optionally none, one or a plurality of additives, and at least one solvent in a defined composition.

12. The method according to any of claims 1 to 11, **characterized in that**
the coating solution (3) is replenished continuously or discontinuously in a defined manner.

13. A device for coating catheters or balloon catheters, **characterized in that**
a. the surrounding device (2) is configured as a ring, tube or helix;
b. **in that** the catheter (1) is fixed in place in a holding device (6) with the aid of a support (5) onto which the catheter (1) is pushed;
c. **in that** the device (2) is moved axially across the catheter (1) a plurality of times;
d. **in that** a heated gas stream (9) which assists in the partial drying between the coating processes and the final drying is generated with a fan (8); and
e. **in that** gas flows are generated at the ends of the catheter (1) with the means for generating a gas flow (7), the gas flows preventing an enrichment of the coating solution and/or a drop formation.

14. The device according to claim 13, **characterized in that**
the enclosing device (2) has a narrow slot.

15. The device according to claim 13 or 14, **characterized in that**
the surrounding device (2) consists of metallic and/or non-metallic materials.

16. The device according to any of claims 13 to 15, **characterized in that**
the surface of the surrounding device (2) is coated and/or is provided with an additional structure.

17. The device according to any of claims 13 to 16, **characterized in that**
the partial drying between the coating processes and the final drying are assisted **in that** the coating takes place in a chamber with an altered pressure.

## Revendications

1. Procédé de revêtement de cathéters ou de cathéters à ballonnet, **caractérisé**
a. **en ce que** le cathéter (1) est entièrement ou pratiquement entièrement enserré de manière radiale avec un écartement constant par un dispositif (2) contenant une quantité mesurée d'une solution de revêtement (3) de telle sorte que
b. la solution de revêtement remplit entièrement l'espace entre le cathéter (1) et le dispositif (2) et enveloppe ainsi le cathéter (1) entièrement de manière radiale sur un tronçon partiel axial,
c. **en ce que** le dispositif (2) est déplacé à plusieurs reprises dans le sens axial sur la surface du cathéter (1), et
d. **en ce qu'**un séchage partiel ciblé de la solution de revêtement (3) appliquée est réalisé entre les opérations de revêtement individuelles.

2. Procédé selon la revendication 1, **caractérisé en ce que**
à l'aide d'un moyen additionnel de production d'un écoulement de gaz (7), un écoulement de gaz est produit à l'encontre du sens d'écoulement de la solution de revêtement (3), lequel empêche un enrichissement de la solution de revêtement (3) et/ou la formation de gouttes aux points d'extrémité du cathéter ou du cathéter à ballonnet (1).

3. Procédé selon la revendication 2, **caractérisé en ce que**
l'écoulement de gaz est composé d'un gaz ou d'un mélange de gaz, est réalisé de manière régulière ou avec des variations dans le temps quant au sens et à l'intensité, et est thermiquement influencé de manière variable dans le temps.

4. Procédé selon l'une des revendications 1 à 3, **caractérisé en ce que**
le séchage partiel entre les opérations de revêtement et le séchage final à la fin du revêtement sont accélérés par réchauffement dans un flux additionnel d'un gaz ou d'un mélange de gaz (9).

5. Procédé selon l'une des revendications 1 à 4, **caractérisé en ce que**
le séchage partiel entre les opérations de revêtement et le séchage final à la fin du revêtement sont accélérés par la réalisation du revêtement dans un espace à pression modifiée.

6. Procédé selon l'une des revendications 1 à 5, **caractérisé en ce que**
le revêtement du cathéter (1) y compris le séchage partiel et complet est répété à plusieurs reprises.

7. Procédé selon l'une des revendications 1 à 5, **caractérisé en ce que**
un cathéter à ballonnet est revêtu, lequel se déploie partiellement par réchauffement avant et/ou pendant l'opération de revêtement.

8. Procédé selon la revendication 7, **caractérisé en ce que**
le réchauffement est réalisé de manière régulière, avec une distribution dans l'espace et/ou une variation dans le temps par réchauffement de l'environnement, par réchauffement du dispositif de retenue, par chaleur rayonnante ou par un flux de gaz réchauffé par l'intermédiaire du cathéter à ballonnet.

9. Procédé selon l'une des revendications 1 à 8, **caractérisé en ce que**
le déplacement axial est réalisé par le déplacement du cathéter (1), par le déplacement du dispositif (2) enserrant ou par le déplacement des deux.

10. Procédé selon l'une des revendications 1 à 9, **caractérisé en ce que**
le cathéter (1) est fixe ou est tourné autour de son axe longitudinal.

11. Procédé selon l'une des revendications 1 à 10, **caractérisé en ce que**
la solution de revêtement (3) contient un agent pharmacologique, au choix aucun, un ou plusieurs additifs, et au moins un solvant dans une composition définie.

12. Procédé selon l'une des revendications 1 à 11, **caractérisé en ce que**
la solution de revêtement (3) est renouvelée de manière continuellement ou discontinuellement définie.

13. Dispositif de revêtement de cathéters ou de cathéters à ballonnet, **caractérisé**
a. **en ce que** le dispositif enserrant (2) est réalisé sous forme d'anneau, de tube ou d'hélice,
b. **en ce que** le cathéter (1) est fixé dans un dispositif de retenue (6) au moyen d'une attache (5) sur laquelle le cathéter (1) est poussé,
c. **en ce que** le dispositif (2) est déplacé axialement sur le cathéter (1) à plusieurs reprises,
d. **en ce qu'**un flux de gaz réchauffé (9) est produit au moyen d'une soufflante (8) et soutient le séchage partiel entre les opérations de revêtement et le séchage final, et
e. **en ce que** des écoulements de gaz sont produits aux extrémités du cathéter (1) par les moyens de production d'un écoulement de gaz (7), lesquels empêchent un enrichissement de la solution de revêtement et/ou la formation de gouttes.

14. Dispositif selon la revendication 13, **caractérisé en ce que**
le dispositif enserrant (2) présente une fente étroite.

15. Dispositif selon la revendication 13 ou 14, **caractérisé en ce que**
le dispositif enserrant (2) est réalisé en matières métalliques et/ou non métalliques.

16. Dispositif selon l'une des revendications 13 à 15, **caractérisé en ce que**
la surface du dispositif enserrant (2) est revêtue et/ou pourvue d'une structure additionnelle.

17. Dispositif selon l'une des revendications 13 à 16, **caractérisé en ce que**
le séchage partiel entre les opérations de revêtement et le séchage final sont soutenus par le fait que le revêtement est effectué dans un espace à pression modifiée.
